# EUROPEAN PATENT SPECIFICATION

(11) **EP 1 645 264 B1**
(45) Date of publication and mention of the grant of the patent: **03.11.2010**
(21) Application number: 04022404.0
(22) Date of filing: 21.09.2004
(51) Int. Cl.: A61K 8/02, A61Q 5/10, A61K 8/41, A61K 8/04

(54) **Product for hair dyeing in a two chamber aerosol can**
Haarfärbezusammensetzung in einer Sprühdose mit zwei getrennten Kammern
Produit pour la coloration des cheveux dans un aérosol à deux chambres

(43) Date of publication of application: 12.04.2006
(73) Proprietor: KPSS-Kao Professional Salon Services GmbH, 64297 Darmstadt (DE)
(72) Inventor: Lamboy, Peter, 64331 Weiterstadt (DE); Vogelsang, Herbert, 64297 Darmstadt-Eberstadt (DE)

(56) References cited:
- EP-A- 0 063 759
- EP-A- 0 269 068
- EP-A- 1 142 562
- WO-A-95/15144
- WO-A-03/091128
- DE-A- 19 717 080
- US-A- 5 775 549

## Description

This invention relates to a process for obtaining an oxidative dyeing product for hair consisting of a two chamber pressurised can and an aqueous composition comprising at least one oxidative dye precursor and optionally at least one coupling agent and optionally at least one direct dye which is mixed with an aqueous composition comprising at least one oxidizing agent prior to application.

Hair dyeing compositions have been marketed for many decades. Especially the oxidative hair dyeing is believed to be producing the most long lasting colours as small uncoloured molecules polymerise partly on hair surface but more importantly inside of hair so that they can not easily been washed away with subsequent hair treatments other than colourations such as shampooing and/or conditioning hair.

These products are usually packed in single dose units because of their sensibility to oxidation when contacting air. Once packaging is opened and emptied partly, remaining product is not anymore stable. This brings certainly high packaging costs and waste, which, in addition, brings about environmental problems.

In order to solve these problems, there have been products available for oxidative hair dyeing packed into two chamber pressurized can which allow partial withdrawal of its content and the remaining part can stably be stored further (for example DE 28 27 610). This type of packaging is first of all not economic from the can material point of view (mostly aluminium) and secondly flammable gas is usually used for building up pressure inside the can between the product and outer can wall. These points bring about other disadvantages such as labelling as flammable and, therefore, special requirements in storage and transportation.

It is highly desirable to achieve a product, especially from the view point of making available multi-portion products, with low cost without loosing the performance in terms of stability and colouring ability during whole life time. Alternative to two-chamber packaging mentioned above, two chamber packaging divided with a movable piston have been made available. This type of pressurized cans have been used especially in food industry and as well as in some cosmetic applications such as shaving gels, shampoo and treatments. So far, there has not been any product made available for oxidative hair colouring packed into a can comprising a movable can inside without having above-mentioned problems.

This invention starts form the problems as explained above and aims primarily for finding an alternative, which is more cost effective, environmental friendly and as well be packed without using flammable gas. The alternative packaging makes withdrawal of the package content in smaller portions than the whole content of the product without affecting the stability of the remaining product during its lifetime. Lifetime of a cosmetic product is in general 3 years after production.

US 5,775,549 and DE 197 17 080 A1 disclose spray package comprising a piston to be used in the fields of foodstuffs, chemistry, cosmetics, dermatology, insecticides and cleaning agents. Nothing is said about an oxidative colouring composition for colouring keratin fibres in both documents.

EP 1 142 562 A1 disclose laccase containing oxidation hair dye composition of one pack type. It is disclosed that the composition can be packed as an aerosol of direct spray type or of dual container type such as piston type. Nothing is disclosed how to obtain an oxidative dyeing product of a dual container type with a piston.

The inventors have surprisingly found out a process for obtaining an oxidative colouring product, wherein
a- the can (1), consisting of an outer wall (2) having a closure (3) with a valve (5) and a bottom (4) with a stopper (6) which contains a vertically movable unsupported piston (7) with a deepened area (10) around the middle of the piston, which fits to the closure area (3 and 5) physically when moved up during emptying the package, the movable piston (7) further divides the whole volume of the can into two completely separate volumes (8 and 9) one (8) being filled with colouring composition and the other (9), being the lower part, is filled with an oxygen free non-flammable gas to build up pressure in the can, is first placed in a filling place,
b- by means of extendable filling nozzle entering the can through the closure area, the movable piston (7) is pushed to the very bottom of the can which is followed by purging the can content with an oxygen free gas, preferably same as the pressurising gas and is selected from nitrogen and carbon dioxide, through a nozzle which may be same or different to the filling nozzle,
c- through the opening (6) at the bottom (4) the lower part of the can is purged with oxygen free gas preferably same as the pressurising gas and is selected from nitrogen and carbon dioxide.
d- pushing the piston (7) down again through the opening with an extendable nozzle and filling the colouring composition already produced into can into the upper part (8),
e- purging the upper space of the can again left after filling the composition with an oxygen free gas,
f- placing the closure and valve on top of the can, and
g- pressurised gas is filled into to lower part (9) and stopper (plug) is placed (6).

The can makes storage of an oxidative colouring composition possible for a long period of time. When the content of such a can is only partially emptied, the remaining is as well stored without showing any signs of instability for a long time. Such kinds of packaging materials are commercially available.

It has as well been found out that in the above described process steps b and c can be exchanged meanings that the step c can be carried out before the step b and then continued with step b and step d etc.

It is the object of the invention to provide product for oxidative colouring hair consisting of a pressurised can as described above and an aqueous composition comprising at least one oxidative dye precursor and optionally at least one coupling agent and optionally at least one direct dye.

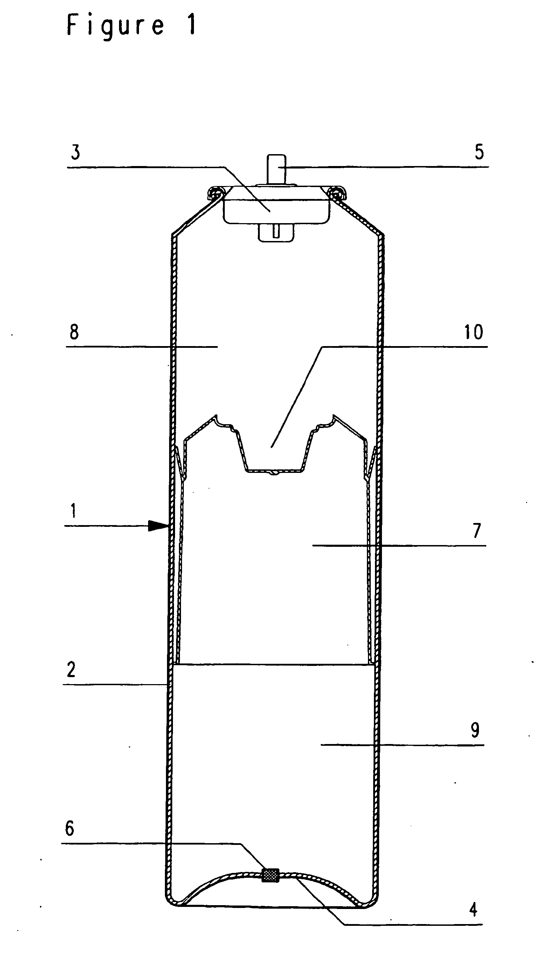

Figure 1 represents schematically pressurised can the numbers correspond to the same as above.

Composition filled into the can is an oxidative dyestuff composition comprising at least one oxidative dyestuff precursor (developing substance) and optionally at least one coupling substance and optionally at least one direct dye. According to the invention, composition can be in the form of emulsion, solution, dispersion and/or gel. Emulsion and gel forms are preferred. The most preferred is emulsion.

The viscosity of the compositions according to the invention ranges from 1,000 to 50,000, in particular 1,000 to 30,000, especially 1,000 to 20,000 mPa.s, measured at 20°C with a Brookfield rotation viscosimeter, with spindle 5 at 5 rpm.

Dyeing composition according to the present invention comprises at least one oxidation dyestuff precursor (developing substance) and optionally at least one coupling substance and optionally at least one direct dye. In principal, it is possible to incorporate developing substances known **per se**. Special mention is made of p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxyethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

The total concentration of the developing substances customarily ranges between about 0.05 % and 5 %, preferably 0.1 % and 4 %, in particular 0.25 % to 0.5 % and 2.5 % to 3 % by weight, calculated to the total hair dyeing composition (excluding the oxidation agent), whereby these figures are always related to the proportion of free base.

The composition according to the present invention contains optionally at least one coupling substance, which can be selected from 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

Further, indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

According to the invention, dyeing composition comprises optionally at least one direct acting dyestuff selected from anionic, cationic and neutral ones. Suitable cationic dyestuffs are in principal those available on the market for hair colouring applications. Some examples to those are:
Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

For this purpose, special reference is made to the PCT application WO 95/15144 of Ciba-Geigy AG. The cationic dyestuffs with the following chemical structures are especially, the most, preferred ones according to the present invention. 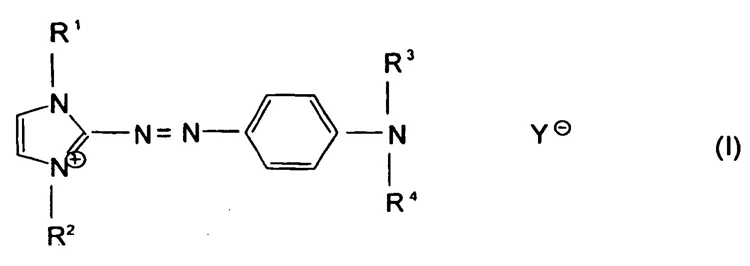 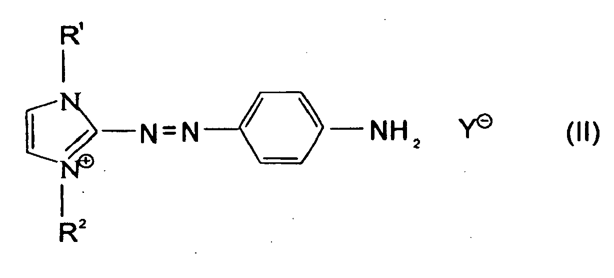 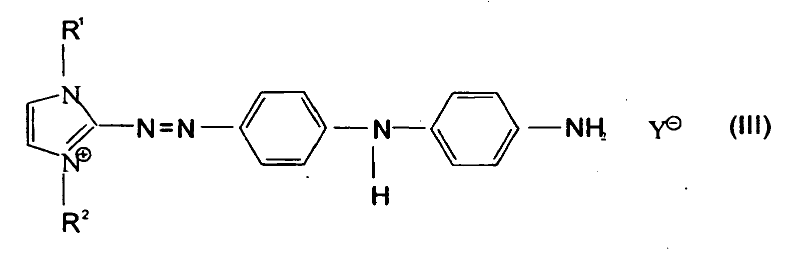 and 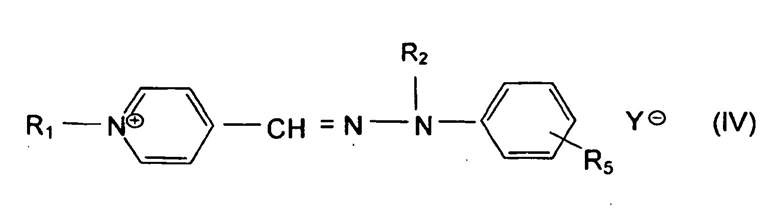 wherein R¹, R², R³ and R⁴ stand for hydrogen, a CH₃- or C₂H₅- group, R⁵ stands for hydrogen, -OCH₃ or -OC₂H₅ and Y is an anion such as chloride, bromide, methosulfate.

The most preferred compounds are the ones according to the formula I, where R₁, R₂, R₃ and R₄ are methyl and Y is chloride, according to formula II where R₁ and R₂ are methyl and Y is chloride and according to the formula IV, where R₁ and R₂, are methyl, R₅ is hydrogen and Y is methosulfate.

Cationic dyestuffs are included into the compositions of the present invention at a concentration of 0.01 to 7.5%, preferably 0.05 to 5% and more preferably 0.05 to 3% by weight calculated to total aqueous composition.

Anionic dyes suitable for the compositions of the present invention are:
Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the most preferred anionic dyestuffs are Acid Red 52, DC Violet 2, DC Red 33, DC Orange 4, DC Red 27 and DC Yellow 10.

According to the invention anionic dyes are included into oxidative dyeing compositions at a concentration of 0.1 to 7.5%, preferably 0.1 to 5%, more preferably 0.1 to 3% by weight calculated to total aqueous composition.

When both anionic and cationic dyes are present in the dyeing compositions the ratio of cationic dyestuffs to acidic dyestuffs by weight is preferably in the range of 3:1 to 1:10, preferably 2: 1 to 1:7 and further more preferably 2:1 to 1:5. In a patent application from the applicant the importance of the ratio of cationic to anionic dyes is disclosed in detail.

Additionally, the coloring compositions of the present invention can comprise neutral nitro dyes (HC dyes), so called nitro dyes as direct dye. Concentration of those can typically be in the range from 0.01 to 2.5%, preferably 0.1 to 2% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

pH of the aqueous composition of the present invention varies between 5 and 12, preferably 6 - 11, more preferably 6.8 to 10. pH of the aqueous composition is adjusted to the required pH by using triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

ln the case that the composition is an emulsion, the most preferred, aqueous composition for oxidative dyeing comprises in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

In the case that the coloring composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 25%, preferably 0.5 to 20% by weight and more preferably 0.5 to 15% by weight calculated to total composition without oxidizing agent.

Coloring compositions according to present invention comprises surfactants selected from anionic, nonionic, amphoteric (or zwiterionic) and/or cationic surfactants as emulsifier or solubilizer. Cationic surfactants are as well used as hair conditioners in the coloring composition.

Anionic surfactants suitable within the scope of the invention are in principal known from the cleansing compositions and are preferably present in an amount from 0.1 to about 10%, preferably 0.2 to 7.5% and most preferably 0.2 - 5% by weight, calculated to the total composition.

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₆- (C₂H₄O)ₙ-O- CH₂COOX,

wherein R₆ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 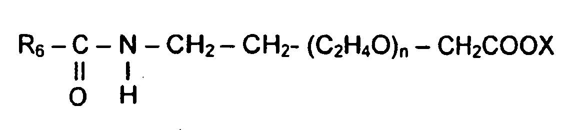 wherein R₆ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof. Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further surfactants in the colouring compositions according to the invention are nonionic surfactants alone or in admixture with anionic surfactants at a weight ratio of 3:1 to 1:3.

These are described as well in Schrader, l.c., on pages 600-601 and pp. 694-695.

Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited are alkyl polyglucosides of the general formula

R₇-O-(R₃O)ₙ-Zₓ,

wherein R₇ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

The concentration of nonionic surfactant is in the range from 0.5 to 15%, preferably 0.5 to 10% by weight and more preferably 0.5 to 7.5% by weight calculated to total composition without oxidizing agent.

As further surfactant component, the colouring compositions according to the invention can also contain amphoteric or zwitterionic surfactants, for example in an amount from about 0.5 % to about 5 %, preferably from about 1 % to about 2.5 % by weight, calculated to the total composition without oxidizing agent.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structures 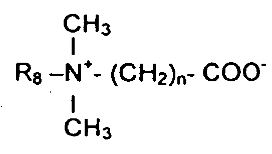 and 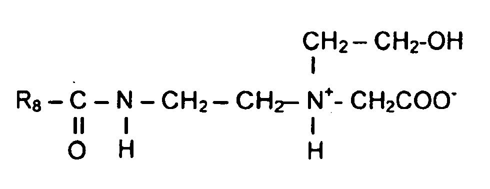 wherein R₈ is a C₈-C₁₈-alkyl group and n is 1 to 3;
sulfobetaines of the structure 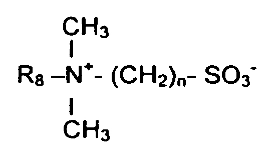 wherein R₈ and n are same as above;
and amidoalkyl betaines of the structure 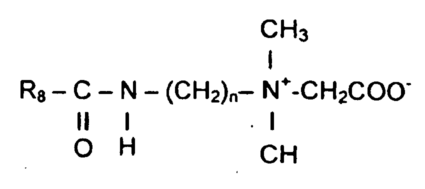 wherein R₈ and n are same as above.

Colouring composition can contain cationic surfactans as emulsifier, solubilizer and/or conditioning ingredients according to the formula, but not limited to. 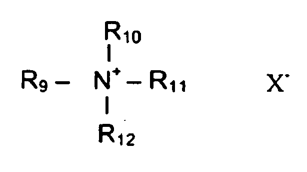 where R₉ is a saturated or unsaturated, branched or non-branched alkyl chain with 8-22 C atoms or

R₁₃ CO NH (CH₂)ₙ

where R₁₃ is saturated or unsaturated, branched or non-branched alkyl chain with 7-21 C atoms and n has typical value of 1 - 4 or

R₁₄ CO O (CH₂)n

where R₁₄ is saturated or unsaturated, branched or non-branched alkyl chain with 7-21 C atoms and n has typical value of 1 - 4, and
R₁₀ is H or unsaturated or saturated, branched or non-branched alkyl chain with 1 - 22 C atoms or

R₁₃ CO NH (CH₂)ₙ

or

R₁₄ CO O (CH₂)ₙ

where R₁₃, R₁₄ and n are same as above.

R₁₁ and R₁₂ are H or lower alkyl chain with 1 to 4 Carbon atoms, and X is typically an anion such as chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Colouring composition can also contain cationic polymers as conditioning agents and gel forming agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.05 - 3% by weight and more preferably 0.05 - 2% by weight calculated to total composition without oxidizing agent.

Colouring composition comprise preferably an organopolysiloxane wherein at least one silicium atom is linked to an alkylene group having a hetero-atom, in particular a nitrogen atom, with a poly-(N-acyl alkyleneimine) units of the formula 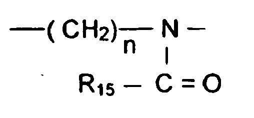 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 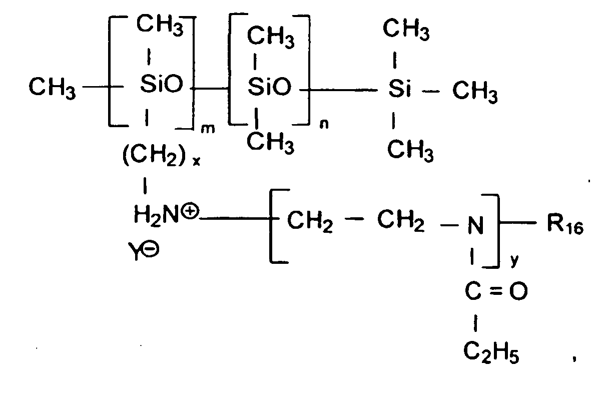 wherein m and n each are numbers from 20 to 10,000, in particular 50 to 7,000, especially 100 to 5,000, x is a number between 1 and 5, preferably 3, and y is a number from 5 to 30, R₁₆ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 25%, preferably 0.5 - 20% by weight and more preferably 0.5 - 15% by weight calculated to the total composition without oxidizing agent.

The hair dyeing compositions according to the invention preferably contain thickening agents, especially in the case a gel type of preparation is preferred. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula X or XI, respectively,

R₁₇ CO (O CH₂ CH₂)ₙ OH formula X

R₁₇ CO (O CH₂ CH₂)ₙ O OC R₁₈ formula XI

where R₁₇ and R₁₈ are independent from each other saturated, unsaturated or branched or non-branched alkyl chain with 7 to 21 C atoms and n is typically 2 - 100.

Another preferred compound in the composition is of ceramide type of compounds according to general formula 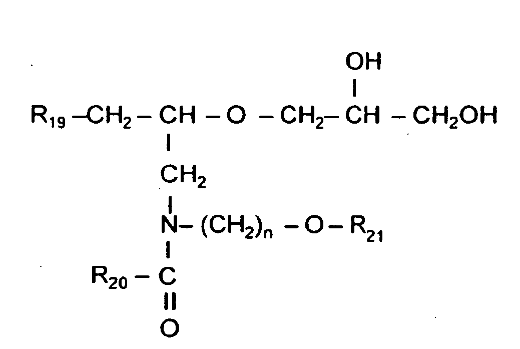 where R₁₉ and R₂₀ are independent from each other alkyl- or alkenyl group mit 10 to 22 carbon atoms, R₂₁ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1 % by weight calculated to the total composition.

The compositions of the present invention can comprise of at least one ubiquinone of the formula (I) 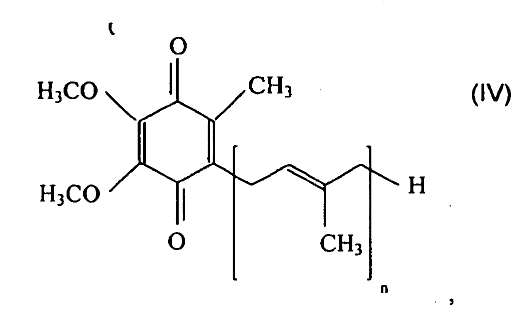 wherein n is a number from 1 to 10. Concentration of ubichinone can vary between 0.001 % and 10 % by weight, calculated to the total composition, excluding the oxidizing agent.

After removal of the content of the product comprising at least one oxidation dyestuff precursor and optionally at least one coupling substance and optionally at least one direct dye, the composition is mixed with an aqueous composition comprising at least one oxidizing agent prior to application onto hair. The oxidizing agents suitable are hydrogen peroxide, urea peroxide, melanin peroxide or perborate salts. The most preferred is hydrogen peroxide, which is used as a lotion containing 3 to 12% by weight, calculated to composition only comprising hydrogen peroxide. The mixing ratio is usually in the range of 4:1 to 1:4, by weight, preferably 3:1 to 1:3 by weight, more preferably 2:1 to 1:3 by weight.

The product according to the present invention comprises at least one gas. Gas is an oxygen free and non-flammable and is selected from nitrogen, carbon dioxide and dinitrogen oxide (N₂O). The pressure within the can should not exceed 15 bar, preferably 10 bar and should be at least 1 bar preferably 3 and more preferably 5 bar bar. The amount of oxygen free, non-flammable gas is determined according to required pressure inside can.

The cans can be emptied with very low level of remains in the can. The emptying is achieved at most not les than 98% of its content by weight.

The following examples are used to illustrate the invention, but not limit it

### Examples

| **Base formulations** | | |
|---|---|---|
| | % by weight I | % by weight II |
| Stearamide MEA | - | 1.50 |
| Cocamide MEA | 4.00 | 2.00 |
| Cetearyl alcohol | 10.00 | 8.00 |
| Tegin P | 1.40 | 1.40 |
| Propylene Glycol | 2.40 | 1.60 |
| Oleic acid | 3.00 | 3.00 |
| Ammonium chloride | 0.50 | 0.50 |
| Tetrasodium EDTA | 0.20 | 0.20 |
| Sodium lauryl sulfate | 1.50 | - |
| Sodium cetearyl sulfate | - | 1.20 |
| Organopolysiloxane A1 of EP 640 643 Ceramide according to formula wherein | 0.20 | 0.20 |
| R₁₈ and R₁₉ are C₁₆ alkyl and R₂₀ is ethyl | 0.10 | 0.20 |
| Water | to 100 | to 100 |

The dyestuffs given in the following as for various color directions are added to the base compositions either I or II. Water amount is reduced in the base formula corresponding to the amount of dyes present in the formulations.
The both base formulations have similar viscosity values of around 5000 mPa. s measured by Brookfield viscosimeter at 20°C with spindle 4.

### Example 1

| | % by weight |
|---|---|
| p-Toluenediamine sulfate | 1.40 |
| Resorcin | 0.60 |
| 3-Aminophenol | 0.10 |
| 2-Amino-4-hydroxyethylaminoanisolsulfate | 0.02 |
| Base formula I | to 100 |

The pH of the composition is adjusted to 9.5. The composition obtained was mixed with a 6% by weight hydrogen peroxide comprising composition at a weight ratio of 1:1 and hair swatches were colored at room temperature for 30 min. A light brown color was obtained.

The composition of example 1 was filled into the can as described above. Nitrogen gas is used to achieve a pressure of 8 bar inside the can. The product was stable over the period of 12 weeks (period tested) at 40°C and at room temperature. No instability is observed in terms of colouring performance on hair, pH, viscosity, appearance (especially macroscopic colour of the composition).

In another run, the filling into the cans was carried out as described above except that the filling the step 3 as described above was not carried out, namely the lower part (9) was not purged with an oxygen free, nonflammable gas and the stability of the product was tested. After storing 2 weeks at 40°C and at room temperature, aqueous composition was found out to be getting darker (macroscopic colour as observed by naked eye) at the places where composition and the piston come into contact showing clearly oxidative instability of the composition, this got even worse during the length of the whole storage test - 12 weeks.

These results show clearly that the above described procedure is very important, especially purging of the lower part (9) is as important as the upper chamber (8) where aqueous composition is filled, in order to guarantee the optimum stability for the whole shelf life.

Similar results are obtained with the following examples as well.

### Example 2

| | % by weight |
|---|---|
| 1-Hydroxyethyl-4,5-diaminopyrazolsulfate | 0.54 |
| 4-Amino-2-hydroxytoluol | 0.27 |
| Base formula no II | to 100 |

The preparation and filling and colouring tests was carried out as above and Red copper colour is obtained.

### Example 3

| | % by weight |
|---|---|
| 2,4,5,6-Tetraaminopyrimidine sulphate | 0.77 |
| 2-Amino-4-hydroxyethylaminoanisolsulfate | 0.90 |
| Base formula I | to 100 |

The preparation and filling and colouring tests was carried out as above and violet colour is obtained.

### Example 4

| | % by weight |
|---|---|
| 2,4,5,6-Tetraaminopyrimidine sulphate | 0.53 |
| 2-Methylresorcin | 0.28 |
| Basic red 51 | 0.20 |
| Base formula I | to 100 |

The preparation and filling and colouring tests was carried out as above and intensive red colour is obtained.

### Example 5

| | % by weight |
|---|---|
| 2,4-Diamino-6-hydroxypyrimidine | 0.28 |
| 4-amino-2hydroxytoluol | 0.27 |
| Base formula II | to 100 |

The preparation and filling and colouring tests was carried out as above and orange colour is obtained.

## Claims

1. Process for obtaining oxidative hair dyeing product consisting of a pressurised can and an aqueous composition **characterised in that**
a- the can (1) consisting of an outer wall (2) having a closure (3) with a valve (5) and a bottom (4) with a stopper (6) which contains a vertically movable unsupported piston (7) with a deepened area (10) around the middle of the piston, which fits to the closure area (3 and 5) physically when moved up during emptying the package, the movable piston (7) further divides the whole volume of the can into two completely separate volumes (8 and 9) one (8) being filled with colouring composition and the other (9), being the lower part, is filled with an oxygen free non-flammable gas to build up pressure in the can, is first placed in a filling place,
b- by means of extendable filling nozzle entering the can through the closure area, the movable piston (7) is pushed to the very bottom of the can which is followed by purging the can content with an oxygen free gas, preferably same as the pressurising gas and is selected from nitrogen and carbon dioxide, through a nozzle which may be same or different to the filling nozzle,
c- through the opening (6) at the bottom (4) the lower part of the can is purged with oxygen free gas preferably same as the pressurising gas and is selected from nitrogen and carbon dioxide,
d- pushing the piston (7) down again through the opening with an extendable nozzle and filling the colouring composition already produced into can into the upper part (8),
e- purging the upper space of the can again left after filling the composition with an oxygen free gas,
f- placing the closure (3) and valve (5) on top of the can, and
g- pressurised gas is filled into to lower part (9) and stopper (plug) (6) is placed.

2. Process according to claim 1 **characterised in that** the steps b and c are carried out in the opposite order.

3. Process according to claims 1 and 2 **characterised in that** oxygen free, non-flammable gas is selected from nitrogen, carbon dioxide and dinitrogen oxide (N₂O).

4. Process according to claims 1 to 3 **characterised in that** the aqueous colouring composition has a viscosity in the range from 1,000 to 50,000 mPa.s measured at 20°C with a Brookfiled viscosimeter with a spindle 5 at 5 rpm.

5. Process according to any of the preceding claims **characterised in that** the aqueous colouring composition comprises at least one coupling substance in addition to oxidative dye precursor.

6. Process according to any of the preceding claims **characterised in that** that the aqueous colouring composition comprises at least one direct dye selected from anionic, cationic and neutral nitro dyes.

7. Process according to claim 5 in that the direct dye is a cationic hair dye.

8. Process according to claim 5 in that the direct dye is neutral nitro hair dye.

9. Process according to any of the preceding claims **characterised in that** the aqueous oxidative dyeing composition is an emulsion and comprises at least one emulgator.

## Patentansprüche

1. Verfahren zur Erstellung von oxidativen Haarfärbeprodukten, bestehend aus einer unter Druck stehenden Dose und einer wässrigen Zusammensetzung, **dadurch gekennzeichnet, dass**
a- die Dose (1), bestehend aus einer äußeren Wand (2), die einen Verschluss (3) mit einem Ventil (5) und einen Boden (4) mit einem Stopfen (6) hat, in der sich ein vertikal beweglicher, nicht unterstützter Kolben (7) mit einer Vertiefung (10) in der Mitte des Kolbens befindet, der weiterhin körperlich in den Verschlussbereich (3 und 5) passt, wenn er sich, während der Entleerung der Verpackung, aufwärts bewegt, der bewegliche Kolben (7) des weiteren das gesamte Volumen der Dose in zwei komplett separate Volumen (8 und 9) trennt, Teil (8) gefüllt mit der färbenden Zusammensetzung und der andere Teil (9), welcher der untere Teil ist, mit einem Sauerstoff freien, nicht brennbaren Gas gefüllt ist, um den Druck in der Dose aufzubauen, die Dose zuerst in einer Füllstation platziert wird und
b- mittels einer einschiebbaren Fülltülle, die durch den Verschlussbereich in die Dose eintritt, der bewegliche Kolben (7) ganz nach unten auf den Boden der Dose gedrückt wird, gefolgt durch Purging (ausblasen) des Dosenvolumens mit einem Sauerstoff freien Gas, vorzugsweise dem gleichen wie das Druck gebende Gas, ausgewählt aus Stickstoff und Kohlendioxid durch eine Tülle, welche die gleiche oder auch eine andere wie die Fülltülle sein kann,
c- durch die Öffnung (6) am Boden (4) der untere Teil der Dose mit einem Sauerstoff freien Gas, vorzugsweise dem gleichen wie das Druck gebende Gas, ausgewählt aus Stickstoff und Kohlendioxid, gepurged (ausgeblasen) wird,
d- der Kolben (7) nochmals mit der einschiebbaren Tülle nach unten gedrückt wird und mit der bereits produzierten färbenden Zusammensetzung die Dose im oberen Teil (8) befüllt wird,
e- der oberste Raum der Dose, der nach dem Befüllen mit der Zusammensetzung verblieben ist, nochmals mit einem Sauerstoff freien Gas gepurged (ausgeblasen) wird,
f- der Verschluss (3) und das Ventil (5) auf den oberen Teil der Dose aufgebracht wird, und
g- das Druck gebende Gas in den unteren Teil (9) gefüllt und der Stopfen (6) eingebracht wird.

2. Verfahren nach Anspruch 1, **dadurch gekennzeichnet, dass** die Schritte b und c in umgekehrter Reihenfolge durchgeführt werden.

3. Verfahren nach den Ansprüchen 1 und 2, **dadurch gekennzeichnet, dass** das Sauerstoff freie, nicht brennbare Gas aus Stickstoff, Kohlendioxid und Distickstoffoxid (N₂O) ausgewählt ist.

4. Verfahren nach den Ansprüchen 1 bis 3, **dadurch gekennzeichnet, dass** die wässrige, färbende Zusammensetzung ein Viskosität im Bereich von 1.000 to 50.000 mPa.s hat, gemessen bei 20°C mit einem Brookfiled Viscosimeter mit einer Spindel 5 bei 5 rpm.

5. Verfahren nach einem der vorhergehenden Ansprüche, **dadurch gekennzeichnet, dass** die wässrige, färbende Zusammensetzung zusätzlich zu einem oxidativen Farbentwickler mindestens eine Kupplersubstanz enthält.

6. Verfahren nach einem der vorhergehenden Ansprüche, **dadurch gekennzeichnet, dass** die wässrige, färbende Zusammensetzung mindestens eine direkt ziehende Farbe, ausgewählt aus anionischen-, kationischen- und neutralen Nitro-Farben enthält.

7. Verfahren nach Anspruch 5, bei dem die direkt ziehende Farbe eine kationische Haarfarbe ist.

8. Verfahren nach Anspruch 5, bei dem die direkt ziehende Farbe eine neutrale Nitro- Haarfarbe ist.

9. Verfahren nach einem der vorhergehenden Ansprüche, **dadurch gekennzeichnet, dass** die wässrige oxidative Farbzusammensetzung eine Emulsion ist und mindestens einen Emulgator enthält.

## Revendications

1. Procédé d'obtention d'un produit de coloration capillaire oxydatif consistant en un générateur d'aérosol sous pression et une composition aqueuse **caractérisé en ce que**
a. le générateur d'aérosol (1) consistant en une paroi extérieure (2) ayant une fermeture (3) avec une valve (5) et un fond (4) avec un obturateur (6) qui contient un piston (7) non supporté déplaçable verticalement avec une zone approfondie (10) autour du milieu du piston, qui s'ajuste en outre à la zone de fermeture (3 et 5) physiquement lorsqu'il est déplacé vers le haut lors du vidage du récipient, le piston mobile (7) divise en outre la totalité du volume du générateur d'aérosol en deux volumes complètement séparés (8 et 9), l'un (8) étant rempli d'une composition colorante et l'autre (9), étant la partie inférieure, est rempli avec un gaz non inflammable dépourvu d'oxygène pour créer une pression dans le générateur d'aérosol, est d'abord placé dans un emplacement de remplissage,
b. au moyen d'une buse de remplissage extensible pénétrant dans le générateur d'aérosol à travers la zone de fermeture, le piston mobile (7) est poussé complètement jusqu'au fond du générateur d'aérosol, ce qui est suivi d'une purge du contenu du générateur d'aérosol avec un gaz dépourvu d'oxygène, préférablement le même que le gaz de pressurisation, et est choisi parmi l'azote et le dioxyde de carbone à travers une buse qui peut être la même que la buse de remplissage ou être différente de celle-ci,
c. à travers l'ouverture (6) dans le fond (4) la partie inférieure du générateur d'aérosol est purgée avec un gaz dépourvu d'oxygène, préférablement le même que le gaz de pressurisation, et est choisi parmi l'azote et le dioxyde de carbone,
d. poussée du piston (7) à nouveau vers le bas à travers l'ouverture avec une buse extensible et remplissage de la composition colorante déjà produite dans le générateur d'aérosol dans la partie supérieure (8),
e. purge de l'espace supérieur du générateur d'aérosol encore laissé après remplissage de la composition avec un gaz dépourvu d'oxygène,
f. mise en place de la fermeture (3) et de la valve (5) sur le dessus du générateur d'aérosol, et
g. un gaz sous pression est rempli dans la partie inférieure (9) et un obturateur (bouchon) (6) est mis en place.

2. Procédé selon la revendication 1 **caractérisé en ce que** les étapes b et c sont exécutées dans l'ordre inverse.

3. Procédé selon les revendications 1 et 2 **caractérisé en ce que** le gaz non inflammable dépourvu d'oxygène est choisi parmi l'azote, le dioxyde de carbone et le monoxyde de diazote (N₂O).

4. Procédé selon les revendications 1 à 3 **caractérisé en ce que** la composition colorante aqueuse a une viscosité dans la plage de 1 000 à 50 000 mPa.s mesurée à 20°C avec un viscosimètre Brookfield équipé d'un mobile 5 à 5 tr/min.

5. Procédé selon l'une quelconque des revendications précédentes **caractérisé en ce que** la composition colorante aqueuse comprend au moins une substance de couplage en plus du précurseur de colorant oxydatif.

6. Procédé selon l'une quelconque des revendications précédentes **caractérisé en ce que** la composition colorante aqueuse comprend au moins un colorant direct choisi parmi des colorants anioniques, cationiques et nitro neutres.

7. Procédé selon la revendication 5, dans lequel le colorant direct est un colorant capillaire cationique.

8. Procédé selon la revendication 5, dans lequel le colorant direct est un colorant capillaire nitro neutre.

9. Procédé selon l'une quelconque des revendications précédentes **caractérisé en ce que** la composition de coloration oxydative aqueuse est une émulsion et comprend au moins un agent émulsionnant.
